# EUROPÄISCHE PATENTANMELDUNG

(11) **EP 2 537 481 A1**
(43) Veröffentlichungstag der Anmeldung: **26.12.2012**
(21) Anmeldenummer: 12168165.4
(22) Anmeldetag: 16.05.2012
(51) Int. Cl.: A61B 18/14, A61B 1/00, A61B 1/307, A61B 18/00

(54) **Arbeitseinsatz für ein endoskopisches Hohlschaftinstrument**

(30) Priorität: 24.06.2011 DE 102011105442
(71) Anmelder: Richard Wolf GmbH, 75438 Knittlingen (DE)
(72) Erfinder: Knodel, Frank, 75438 Knittlingen (DE); Bartolic, Josef, 76135 Karlsruhe (DE); Körner, Dipl.-Ing. Eberhard, 75438 Knittlingen (DE)
(74) Vertreter: Patentanwälte Vollmann & Hemmer

(57) **Zusammenfassung**

Ein Arbeitseinsatz für ein endoskopisches Hohlschaftinstrument weist einen Schaft (2) auf, an dessen Außenseite eine relativ zu dem Schaft (2) axial verschiebbare monopolare Arbeitselektrode (10) angeordnet ist. Die Arbeitselektrode (10) ist mit dem Schaft (2) an einem distalen Endabschnitt des Schaftes (2) über ein Führungsbauteil (26a), das eine elektrische Isolierung zwischen der Arbeitselektrode (10) und dem Schaft (2) bildet, verbunden. (Fig. 2)

## Beschreibung

Die Erfindung betrifft einen Arbeitseinsatz für ein endoskopisches Hohlschaftinstrument mit den im Oberbegriff des Anspruchs 1 angegebenen Merkmalen.

Bei einer Vielzahl medizinischer Therapieverfahren wird die monopolare HF-Applikationstechnik eingesetzt. Hierbei werden monopolare Arbeitselektroden zur Resektion und Vaporisation von Körpergewebe sowie zur Koagulation verwendet. Eine solche Arbeitselektrode ist üblicherweise Teil eines Arbeitseinsatzes mit einer in einem metallischen Schaft angeordneten Beobachtungsoptik und wird über ein endoskopisches Hohlschaftinstrument einem Behandlungsort zugeführt. Die Arbeitselektrode ist bis auf ihr zur Applikation dienendes distales Ende mit einer elektrisch isolierenden Ummantelung umgeben.

Neben Arbeitseinsätzen, bei denen die drahtförmige Arbeitselektrode entlang des Schaftes für die Beobachtungsoptik geführt ist, sind auch solche Arbeitseinsätze bekannt, bei denen die Arbeitselektrode außenseitig des Schaftes für die Beobachtungsoptik angeordnet ist. Insbesondere bei letztgenannten Arbeitseinsätzen besteht aufgrund unsachgemäßer Bedienung oder einer beschädigten elektrischen Isolierung der Arbeitselektrode die Gefahr, dass es zu einem Kurzschluss zwischen der Arbeitselektrode und dem Schaft für die Beobachtungsoptik oder dem metallischen Schaft des Hohlschaftinstruments kommt.

Die Aufgabe der Erfindung ist es, einen Arbeitseinsatz der oben genannten Art mit einer außenseitig eines Schaftes für eine Beobachtungsoptik angeordneten Arbeitselektrode zu schaffen, der gegenüber den bislang bekannten Arbeitseinsätzen einen besseren Schutz vor einer elektrischen Kurzschlussbildung bietet.

Gelöst wird diese Aufgabe durch einen Arbeitseinsatz für ein endoskopisches Hohlschaftinstrument mit den in Anspruch 1 angegebenen Merkmalen. Vorteilhafte Weiterbildungen dieses Arbeitseinsatzes ergeben sich aus den Unteransprüchen, der nachfolgenden Beschreibung sowie der Zeichnung. Hierbei können gemäß der Erfindung die in den Unteransprüchen angegebenen Merkmale jeweils für sich aber auch in technisch sinnvoller Kombination die erfindungsgemäße Lösung gemäß Anspruch 1 weiter ausgestalten.

Der erfindungsgemäße Arbeitseinsatz für ein endoskopisches Hohlschaftinstrument weist einen Schaft auf. In diesem Schaft sind ein elektronisches oder optisches System und zweckmäßigerweise entsprechende Beleuchtungsmittel, wie bspw. Lichtleitfasern oder LEDs angeordnet. Der Schaft wird von einem geraden metallischen Rohr gebildet. An der Außenseite des Schaftes ist eine relativ zu dem Schaft axial verschiebbare HF-Elektrode als Arbeitselektrode angeordnet. Hierbei kann es sich bspw. um eine zur Resektion, Vaporisation oder Koagulation verwendete Elektrode handeln. Die Arbeitselektrode ist drahtförmig ausgebildet und erstreckt sich im Wesentlichen über die gesamte Länge des Schaftes parallel zu dessen Längsachse. An ihrem proximalen Ende ist die Arbeitselektrode an einen HF-Stromanschluss angeschlossen. Bis auf einen distalen Endbereich ist die Arbeitselektrode mit einer elektrischen Isolierung ummantelt. Zur Stabilisierung der Arbeitselektrode ist diese mit dem Schaft an einem distalen Endabschnitt des Schaftes über ein Führungsbauteil verbunden. Das Führungsbauteil ist zweckmäßigerweise derart ausgebildet, dass es eine Bewegung der Arbeitselektrode relativ zu dem Schaft gestattet, d. h. die Arbeitselektrode kann entlang des Schaftes axial verschoben werden.

Gemäß der Erfindung bildet das Führungsbauteil eine elektrische Isolierung zwischen der Arbeitselektrode und dem Schaft für das optische System. Neben der elektrisch isolierenden Ummantelung der Arbeitselektrode wird demnach mit dem Führungsbauteil ein weiterer elektrischer Isolator zwischen der Arbeitselektrode und dem Schaft zur Verfügung gestellt, wodurch ein unerwünschter Stromfluss von der spannungsführenden Arbeitselektrode zu dem elektrisch leitenden Schaft des optischen Systems im Arbeitseinsatz zusätzlich wirksam verhindert wird. Selbst wenn die Isolierummantelung der Arbeitselektrode im Bereich des Führungsbauteils eine Beschädigung aufweisen sollte, wird ein unerwünschter Stromfluss bzw. Kurzschluss zwischen Arbeitselektrode und Schaft verhindert.

Zur Bildung eines elektrischen Isolators ist zumindest ein Abschnitt des Führungsbauteils aus einem elektrischen Isolierstoff, d.h. aus einem elektrisch nicht leitenden Material ausgebildet. Dieser Abschnitt ist typischerweise so angeordnet, dass er eine elektrisch leitende Verbindung von der Arbeitselektrode zu dem Schaft verhindert. Bevorzugt ist allerdings vorgesehen, dass das Führungsbauteil vollständig aus einem elektrisch nicht leitenden Material ausgebildet ist. Hierbei kann das Führungsbauteil grundsätzlich aus allen elektrischen Isolierstoffen bestehen. Bevorzugt ist das Führungsbauteil aber aus Kunststoff ausgebildet. Die Verwendung von Kunststoff als Material für das Führungsbauteil ist insbesondere dann von Vorteil, wenn das Führungsbauteil bewegbar an dem Schaft befestigt ist. Hier ermöglicht der Kunststoff eine vergleichsweise reibungsarme Bewegung des Führungsbauteils auf dem Schaft. Besonders wirtschaftlich kann das Führungsbauteil mittels eines Spritzgussverfahrens hergestellt werden. Das Führungsbauteil ist also vorzugsweise ein Kunststoffspritzgussteil.

Bei einer weiteren bevorzugten Ausgestaltung des erfindungsgemäßen Arbeitseinsatzes ist das Führungsbauteil fest mit der Arbeitselektrode verbunden. Dementsprechend bilden das Führungsbauteil und die Arbeitselektrode eine starre Einheit. Bei dieser Ausgestaltung ist das Führungsbauteil bewegbar mit dem Schaft des optischen Systems und dem Arbeitseinsatz verbunden, d. h., das Führungsbauteil ist auf dem Schaft in Längsrichtung des Schaftes verschiebbar.

Vorteilhaft ist vorgesehen, dass das Führungsbauteil den Schaft zumindest teilweise umgreift. Hierbei ist es zur Befestigung des Führungsbauteils an dem Schaft erforderlich, dass das Führungsbauteil den Schaft um mehr als den halben Schaftumfang umgreift. Ein zur Befestigung des Führungsbauteils an dem Schaft dienender Abschnitt des Führungsbauteils kann vorzugsweise gabelförmig ausgebildet sein und zwei eine Gabel bildende Schenkel aufweisen, die sich um den Schaft in einem Winkelbereich von mehr als 180° herum erstrecken.

Daneben kann das Führungsbauteil auch derart ausgebildet sein, dass es den Schaft vollständig umgreift. In diesem Zusammenhang ist vorteilhaft eine Ausgestaltung des Führungsbauteils vorgesehen, bei der an dem Führungsbauteil ein rohrförmiger Führungskanal zur Aufnahme des Schaftes ausgebildet ist. Demzufolge weist das Führungsbauteil eine offene oder geschlossene Führung auf, deren Querschnitt mit dem Außenquerschnitt des Schaftes des optischen Systems korrespondiert. Durch diese Führung ist das optische System auf dem Schaft mit geringem radialen Spiel geführt.

Zweckmäßigerweise weist der erfindungsgemäße Arbeitseinsatz eine proximalseitig angeordnete Handhabe auf. Mit dieser Handhabe ist die Arbeitselektrode relativ zu dem Schaft verschiebbar. Hierzu ist die Arbeitselektrode mit der Handhabe lösbar gekoppelt und kann durch Verschieben in distaler und in proximaler Richtung auf dem Schaft des optischen Systems bewegt werden. Um auch proximal eine stabile Führung der Arbeitselektrode außenseitig des Schaftes sicherzustellen, weist die Handhabe an ihrem distalen Ende eine vorzugsweise metallische parallel zum Schaft verlaufende rohrförmige Führung für die Arbeitselektrode auf. Diese Führung für die Arbeitselektrode ist radial außenseitig des Schaftes des Arbeitseinsatzes angeordnet und mit dem Schaft verbunden.

Vorteilhaft ist die Arbeitselektrode direkt proximalseitig des Führungsbauteils von einem Verstärkungsrohr umgeben. Das Verstärkungsrohr erstreckt sich demnach direkt in Richtung der an der Handhabe ausgebildeten Führung für die Arbeitselektrode. Auch das vorzugsweise metallisch ausgebildete Verstärkungsrohr dient zur Stabilisierung der Arbeitselektrode und ist innerhalb oder entlang des Schaftes des Arbeitseinsatzes in distaler und proximaler Richtung mit der Arbeitselektrode verschiebbar.

Zweckmäßigerweise ist die Länge des Verstärkungsrohrs derart bemessen, dass es bei der Verschiebung der Arbeitselektrode die handhabeseitig ausgebildete Führung für die Arbeitselektrode nicht kontaktieren kann. Dementsprechend besteht zwischen dem Verstärkungsrohr und der an der Handhabe ausgebildeten Führung für die Arbeitselektrode auch dann ein Abstand, wenn die Arbeitselektrode mit dem Verstärkungsrohr in größtmöglicher Weise proximal verschoben ist. Dieser immer bestehende Abstand zwischen Verstärkungsrohr und handhabeseitiger Führung hat den Vorteil, dass es bei einer Beschädigung der Isolierummantelung der Arbeitselektrode im Bereich des Führungsbauteils oder des Verstärkungsrohrs keinen Stromübergang von dem metallischen Verstärkungsrohr auf die ebenfalls metallisch ausgebildete handhabeseitige Führung und von dort auf die Handhabe geben kann. Zusätzlich resultiert durch die gewählte kurze Länge des Verstärkungsrohres einerseits und des elektrisch isolierenden Führungsbauteils andererseits der Vorteil, dass die gesamte Kapazität des Resektoskopes und des daraus resultierenden Arbeitsstromes reduziert werden.

Vorteilhafterweise wird bei dem erfindungsgemäßen Arbeitseinsatz angestrebt, den Schaft und die damit verbundene Arbeitselektrode stabil in dem Hohlschaftinstrument zu führen, über das der Arbeitseinsatz zu dem im Körperinneren liegenden Behandlungsgebiet geführt wird. Zu diesem Zweck ist bevorzugt an dem Führungsbauteil ein radial auskragender Vorsprung ausgebildet. Dieser Vorsprung bildet vorteilhaft eine Anlagefläche an die Innenwandung des Hohlschafts des endoskopischen Hohlschaftinstruments. Die Abmessung des Vorsprungs quer zur Längsachse des Schaftes des Arbeitseinsatzes ist zweckmäßigerweise derart, dass der Schaft des Arbeitseinsatzes mit dem an dem Schaft befestigen Führungsbauteil für die Arbeitselektrode in dem Hohlschaft, in dem der Arbeitseinsatz eingeführt ist, mit geringem radialen Spiel geführt ist. D. h. der Arbeitseinsatz kann in dem Hohlschaft zwar in Längsrichtung des Hohlschafts ungehindert bewegt werden, Bewegung des Arbeitseinsatzes quer zur Längsausdehnung des Hohlschafts sind aber nur in einem zu vernachlässigenden Maße möglich.

Besonders vorteilhaft können mehrere Führungsbauteile bereitgestellt werden, deren daran ausgebildeter Vorsprung sich hinsichtlich der Abmessungen unterscheidet, sodass der Arbeitseinsatz bei Auswahl des geeigneten Vorsprungs in Hohlschäften mit unterschiedlichen Innendurchmessern in der oben beschrieben Weise geführt wird. Daneben können vorteilhaft solche Führungsbauteile zur Verfügung gestellt werden, deren Anlageflächen in Abhängigkeit des jeweiligen Hohlschaftes an die Innenwandung dieser Hohlschäfte durch unterschiedlich ausgebildete Vorsprünge angepasst sind.

Weiter vorteilhaft ist vorgesehen, dass der Arbeitseinsatz eine optische Markierung aufweist. Diese optische Markierung kann eine Kennzeichnung darstellen, die z.B. die Elektrodenausführung, die Eignung des Führungsbauteils zur Befestigung an einem Schaft mit einem bestimmten Schaftdurchmesser oder die Eignung eines Arbeitseinsatzes zum Einsatz in einem Hohlschaft eines Hohlschaftinstruments mit einem bestimmten Innenquerschnitt kenntlich macht. Über unterschiedliche optische Markierungen ist es in einfacher Weise möglich, eine erforderliche Arbeitselektrode bzw. einen erforderlichen Arbeitseinsatz auszuwählen. Bei der optischen Markierung handelt es sich bevorzugt um eine Farbmarkierung. Besonders einfach können Führungsbauteile, die aus Kunststoff ausgebildet sind, aus farblich unterschiedlichen Kunststoffen hergestellt werden.

Nachfolgend ist die Erfindung anhand von in der Zeichnung dargestellten Ausführungsbeispielen näher erläutert. In der Zeichnung zeigt:

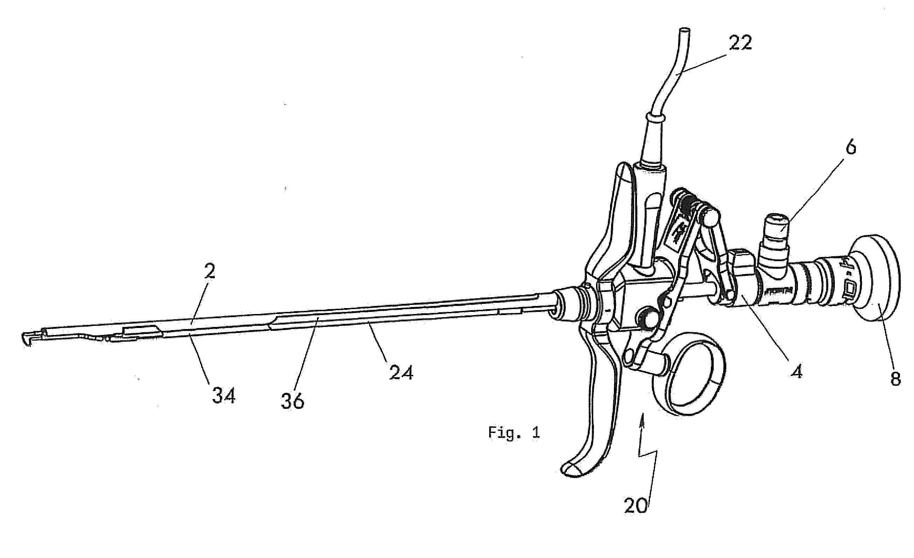

- Fig. 1: einen Arbeitseinsatz für ein endoskopisches Hohlschaftinstrument in schematisch vereinfachter perspektivischer Darstellung,
- Fig. 2: schematisch vereinfacht einen distalen Endabschnitt des Arbeitseinsatzes nach Fig. 1 in einer ersten Ausführungsform,
- Fig. 3: schematisch vereinfacht einen distalen Endabschnitt des Arbeitseinsatzes nach Fig. 1 in einer zweiten Ausführungsform und
- Fig. 4: schematisch vereinfacht in einer Schnittansicht einen distalen Endabschnitt eines endoskopischen Hohlschaftinstrumentes mit einem darin angeordneten Arbeitseinsatz nach Fig. 1.

Der dargestellte Arbeitseinsatz weist eine Optik mit einem äußeren Schaft 2 auf, der von einem geraden, starren, metallischen Rohr gebildet wird. In dem Schaft 2 sind ein elektronisches oder optisches System zum Beobachten eines Behandlungsgebiets und Beleuchtungsmittel zum Beleuchten des Behandlungsgebiets angeordnet. An dem proximalen Ende des Arbeitseinsatzes ist ein Kupplungsteil 4 zur Aufnahme einer Optik angeordnet.

An der Außenseite des Schaftes 2 ist eine monopolare Arbeitselektrode 10 angeordnet. Die Arbeitselektrode 10 ist parallel zu dem Schaft 2 ausgerichtet. Bei der dargestellten Arbeitselektrode 10 handelt es sich um eine Schneidelektrode. Die Arbeitselektrode 10 weist einen Elektrodenstiel 12 auf, der sich distalseitig in zwei voneinander beabstandete Elektrodenschenkel 14 und 16 gabelt. Von dem distalen Ende des Elektrodenschenkels 14 führt ein durch den Elektrodenstiel 12 und die Elektrodenschenkel 14 und 16 geführter Elektrodendraht 18 zu dem distalen Ende des Elektrodenschenkels 16, wobei er zwischen den distalen Enden der Elektrodenschenkel 14 und 16 frei liegt und so eine Schneidschlinge bildet. Sowohl im Bereich des Elektrodenstiels 12 als auch im Bereich der Elektrodenschenkel 14 und 16 weist die Arbeitselektrode 10 außenseitig eine elektrische Isolierummantelung auf.

Proximalseitig ist die Arbeitselektrode 10 mit einer Handhabe 20 gekoppelt und mit einem an der Handhabe 20 angeordneten Hochfrequenz-Stromanschluss 22 verbunden. Durch die Handhabe 20 ist darüber hinaus das optische System mit ihrem Schaft 2 geführt. Mittels der Handhabe 20 ist die Arbeitselektrode 10 entlang des Schaftes 2 in distaler und proximaler Richtung verschiebbar.

An dem distalen Ende der Handhabe 20 ist eine rohrförmige metallische Führung 24 für die Arbeitselektrode 10 ausgebildet. Diese Führung 24 bildet einen Führungskanal durch den die Arbeitselektrode 10 hindurchgeführt ist. Des Weiteren ist die Führung 24 mit dem Schaft 2 verbunden.

In dem Bereich der Arbeitselektrode 10, in dem der Elektrodenstiel 12 in die beiden Elektrodenschenkel 14 und 16 übergeht, ist an dem Elektrodenstiel 12 ein Führungsbauteil 26a bzw. 26b starr befestigt. Hierbei ist die Arbeitselektrode 10 bzw. deren Elektrodenstiel 12 durch einen an dem Führungsbauteil 26a, 26b ausgebildeten Kanal geführt, der den Elektrodenstiel 12 umfänglich umgibt. Zur Führung der Arbeitselektrode 10 an dem Schaft 2 ist das Führungsbauteil 26a bzw. 26b mit dem Schaft 2 des optischen Systems verbunden, wobei die Verbindung von Führungsbauteil 26a bzw. 26b und Schaft 2 derart ist, dass das Führungsbauteil 26a bzw. 26b zwar in Richtung quer zu dem Schaft 2 formschlüssig festgelegt ist aber in Längsrichtung des Schaftes 2 bewegbar ist.

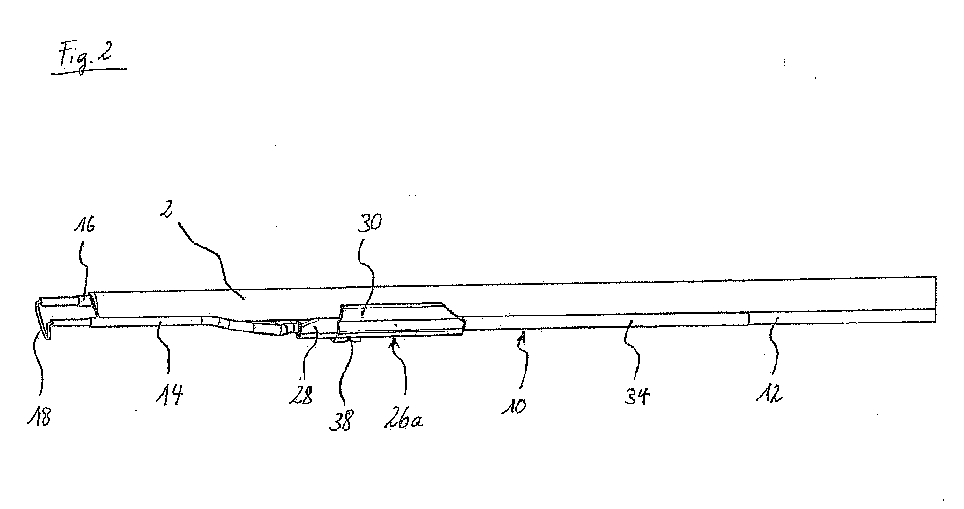

Bei dem in Fig. 2 dargestellten Ausführungsbeispiel weist das Führungsbauteil 26a außenseitig eines Bereichs 28, durch den die Arbeitselektrode 10 geführt ist, zwei seitlich auskragende Schenkel 30 auf, die den Schaft 2 an zwei gegenüberliegenden Seiten des Schaftes 2 teilweise umgreifen.

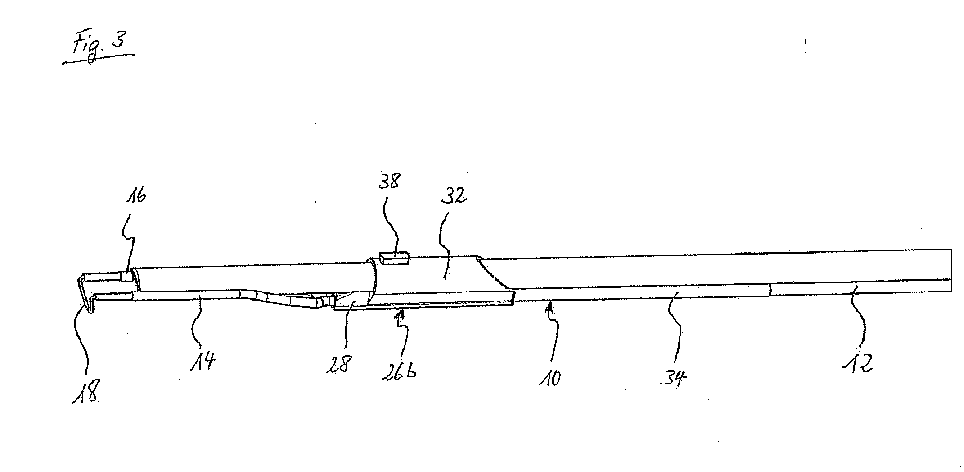

Das in Fig. 3 dargestellte Führungsbauteil 26b umgreift den Schaft 2 vollständig. Hier ist außenseitig des Bereichs 28 des Führungsbauteils 26b ein Bereich 32 vorgesehen, der einen geschlossenen Führungskanal für den Schaft 2 bildet, so dass der Schaft 2 umfänglich vollständig von dem Führungsbauteil 26b umgeben ist.

Sowohl das Führungsbauteil 26a als auch das Führungsbauteil 26b sind als Kunststoffspritzgussteil ausgebildet, also als elektrisch nicht leitend anzusehen. Dementsprechend bilden die Führungsbauteile 26a und 26b elektrische Isolatoren, die den Arbeitseinsatz bei einer Beschädigung der Isolierummantelung der Arbeitselektrode 10 im Bereich des Führungsbauteils 26a bzw. 26b vor einem unerwünschten Stromübergang von der Arbeitselektrode 10 zu dem einen elektrischen Leiter bildenden Schaft 2 schützen.

Proximalseitig der Führungsbauteile 26a und 26b ist der Elektrodenstiel 12 jeweils von einem Verstärkungsrohr 34 umgeben. Das Verstärkungsrohr 34, das mit der Arbeitselektrode 10 fest verbunden ist und dementsprechend mit der Arbeitselektrode 10 in distaler und proximaler Richtung verschiebbar ist, dient zur Stabilisierung der Arbeitselektrode 10 und ist metallisch, also elektrisch leitend, ausgebildet. Die Länge des Verstärkungsrohrs 34 ist so bemessen, dass es bei seiner Verschiebung mit der Arbeitselektrode 10 in Richtung der Handhabe 20 nicht die Führung 24 der Handhabe 20 kontaktieren kann. D. h. zwischen dem Verstärkungsrohr 34 und der Führung 24 besteht immer ein Bereich 36, in dem der mit einer elektrischen Isolierung umgebenen Elektrodenstiel 12 freiliegt. Bei einer Beschädigung der elektrischen Isolierung des Elektrodenstiels 12 innerhalb des Verstärkungsrohrs 34 kann es demnach nicht zu einem durch das Verschieben des Verstärkungsrohrs 34 verursachten Stromübergang von dem Verstärkungsrohr 34 auf die Führung 24 und die sich daran proximal anschließende Handhabe 20 kommen.

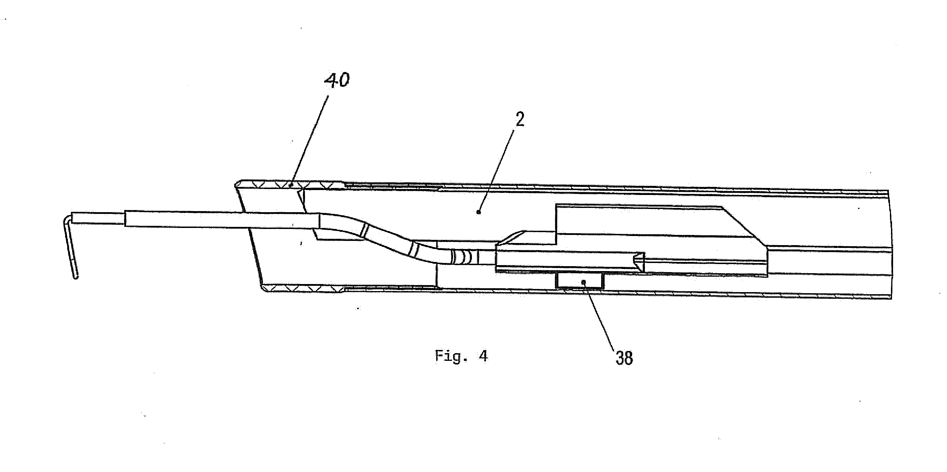

Sowohl das Führungsbauteil 26a als auch das Führungsbauteil 26b weisen jeweils einen radial nach außen kragenden Vorsprung 38 auf. Bei dem in Fig. 2 dargestellten Führungsbauteil 26a ist dieser Vorsprung 38 außenseitig des Bereichs 28 angeordnet, während der Vorsprung 38 bei dem in Fig. 3 dargestellten Ausführungsbeispiel an der Außenseite des Bereichs 32 angeordnet ist. Wie aus Fig. 4 hervorgeht, bildet die Außenseite des Vorsprungs 38 eine Anlagefläche an die Innenwandung eines Hohlschafts 40 eines endoskopischen Hohlschaftsinstruments, über den der Arbeitseinsatz zu einem Behandlungsgebiet geführt wird. Die Höhe des Vorsprungs 38 ist jeweils korrespondierend zu dem Innendurchmesser des Hohlschaftes 40 gewählt, in den der Arbeitseinsatz eingesetzt werden soll, derart, dass der Schaft 2 und das damit außenseitig verbundene Führungsbauteil 26a bzw. 26b in dem Hohlschaft 40 nur ein sehr geringes radiales Spiel zu der Innenwandung des Hohlschaftes 40 aufweisen.

Aus der Zeichnung nicht ersichtlich weisen die Führungsbauteile 26a und 26b jeweils eine Farbmarkierung auf, mit der z.B. eine spezielle Elektrodenausführung oder die Eignung für einen Hohlschaft eines endoskopischen Hohlschaftinstrumentes mit einem bestimmten Innendurchmesser direkt kenntlich gemacht wird.

### Bezugszeichenliste

- 2 -: Schaft
- 4 -: Kupplungsteil
- 6 -: Lichtleiteranschluss
- 8 -: Okular
- 10 -: Arbeitselektrode
- 12 -: Elektrodenstiel
- 14 -: Elektrodenschenkel
- 16 -: Elektrodenschenkel
- 18 -: Elektrodendraht
- 20 -: Handhabe
- 22 -: Hochfrequenz-Stromanschluss
- 24 -: Führung
- 26 -: Führungsbauteil
- 28 -: Bereich
- 30 -: Schenkel
- 32 -: Bereich
- 34 -: Verstärkungsrohr
- 36 -: Bereich
- 38 -: Vorsprung
- 40 -: Hohlschaft

## Patentansprüche

1. Arbeitseinsatz für ein endoskopisches Hohlschaftinstrument, mit einem Schaft (2), an dessen Außenseite eine relativ zu dem Schaft (2) axial verschiebbare Arbeitselektrode (10) angeordnet ist, wobei die Arbeitselektrode (10) mit dem Schaft (2) an einem distalen Endabschnitt des Schafts (2) über ein Führungsbauteil (26a, 26b) verbunden ist, **dadurch gekennzeichnet, dass** das Führungsbauteil (26a, 26b) eine elektrische Isolierung zwischen der Arbeitselektrode (10) und dem Schaft (2) bildet.

2. Arbeitseinsatz nach Anspruch 1, **dadurch gekennzeichnet, dass** das Führungsbauteil (26a, 26b) aus einem elektrisch nicht leitenden Material ausgebildet ist.

3. Arbeitseinsatz nach einem der vorangehenden Ansprüche, **dadurch gekennzeichnet, dass** das Führungsbauteil (26a, 26b) aus Kunststoff besteht.

4. Arbeitseinsatz nach einem der vorangehenden Ansprüche, **dadurch gekennzeichnet, dass** das Führungsbauteil (26a, 26b) fest mit derArbeitselektrode (10) verbunden ist.

5. Arbeitseinsatz nach einem der vorangehenden Ansprüche, **dadurch gekennzeichnet, dass** das Führungsbauteil (26a, 26b) den Schaft (2) zumindest teilweise umfänglich umgreift.

6. Arbeitseinsatz nach einem der vorangehenden Ansprüche, **dadurch gekennzeichnet, dass** an dem Führungsbauteil (26b) ein rohrförmiger Führungskanal zur Aufnahme des Schaftes (2) des optischen Systems ausgebildet ist.

7. Arbeitseinsatz nach einem der vorangehenden Ansprüche, **dadurch gekennzeichnet, dass** der Arbeitseinsatz eine proximalseitig angeordnete Handhabe (20) aufweist, mit welcher die Arbeitselektrode (10) verschiebbar ist, wobei die Handhabe (20) an ihrem distalen Ende eine rohrförmige Führung (24) für die Arbeitselektrode (10) aufweist.

8. Arbeitseinsatz nach Anspruch 7, **dadurch gekennzeichnet, dass** die Arbeitselektrode (10) direkt proximalseitig des Führungsbauteils (26a, 26b) von einem Verstärkungsrohr (34) umgeben ist, dessen Länge derart bemessen ist, dass es bei der Verschiebung der Arbeitselektrode (10) die handhabeseitig ausgebildete Führung (24) für die Arbeitselektrode (10) nicht kontaktiert.

9. Arbeitseinsatz nach einem der vorangehenden Ansprüche, **dadurch gekennzeichnet, dass** an dem Führungsbauteil (26a, 26b) ein radial auskragender Vorsprung (38) ausgebildet ist, welcher eine Anlagefläche an die Innenwandung des Hohlschafts (40) des endoskopischen Hohlschaftinstruments bildet.

10. Arbeitseinsatz nach Anspruch 9, **dadurch gekennzeichnet, dass** der Arbeitseinsatz eine optische Markierung aufweist.
